# EUROPEAN PATENT SPECIFICATION

(11) **EP 2 150 626 B1**
(45) Date of publication and mention of the grant of the patent: **30.09.2015**
(21) Application number: 08748039.8
(22) Date of filing: 06.06.2008
(51) Int. Cl.: C12Q 1/68

(54) **EPIGENETIC METHODS**
EPIGENETISCHE VERFAHREN
MÉTHODES ÉPIGÉNÉTIQUES

(30) Priority: 07.06.2007 AU 2007903075 P
(43) Date of publication of application: 10.02.2010
(73) Proprietor: Haplomic Technologies Pty Ltd., St Kilda West, VIC 3182 (AU)
(72) Inventor: SIMONS, Malcolm, James, Bell Post Hill, Victoria 3215 (AU)
(74) Representative: Gibson, Mark
(86) International application number: PCT/AU2008/000806
(87) International publication number: WO 2008/148159

(56) References cited:
- WO-A2-2008/009365
- MURPHY S K ET AL: "Epigenetic detection of human chromosome 14 uniparental disomy." HUMAN MUTATION JUL 2003 LNKD- PUBMED:12815599, vol. 22, no. 1, July 2003 (2003-07), pages 92-97, XP008041216 ISSN: 1098-1004
- KIM Y ET AL: "Detection of maternal uniparental disomy at the two imprinted genes on chromosome 7, GRB10 and PEG1/MEST, in a Silver-Russell syndrome patient using methylation-specific PCR assays." CLINICAL GENETICS MAR 2005 LNKD- PUBMED:15691366, vol. 67, no. 3, March 2005 (2005-03), pages 267-269, XP007914315 ISSN: 0009-9163
- PROCTER MELINDA ET AL: "Molecular diagnosis of Prader-Willi and Angelman syndromes by methylation-specific melting analysis and methylation-specific multiplex ligation-dependent probe amplification." CLINICAL CHEMISTRY JUL 2006 LNKD- PUBMED:16690734, vol. 52, no. 7, July 2006 (2006-07), pages 1276-1283, XP007914313 ISSN: 0009-9147
- EGGERMANN T ET AL: "Uniparental disomy: Clinical indications for testing in growth retardation" 20020601; 20020600, vol. 161, no. 6, 1 June 2002 (2002-06-01), pages 305-312, XP002328256
- LALANDE M: "Parental imprinting and human disease" ANNUAL REVIEW OF GENETICS, ANNUAL REVIEWS INC., PALO ALTO, CA, US LNKD- DOI:10.1146/ANNUREV.GENET.30.1.173, vol. 30, 1 January 1996 (1996-01-01), pages 173-195, XP002328257 ISSN: 0066-4197
- CARR M.S. ET AL.: 'Allele-specific histone modifications regulate expression of the Dlk1-Gtl2 imprinted domain' GENOMICS vol. 89, no. 2, 2007, pages 280 - 290, XP005762501
- EL-MAARRI O. ET AL.: 'Maternal alleles acquiring paternal methylation patterns in biparental complete hydatidiform moles' HUMAN MOLECULAR GENETICS vol. 12, no. 12, 2003, pages 1405 - 1413, XP008124792
- SASAMOTO H. ET AL.: 'Allele-specific methylation analysis on upstream promoter region of H19 by methylation-specific PCR with confronting two-pair primers' INTERNATIONAL JOURNAL OF ONCOLOGY vol. 25, no. 5, 2004, pages 1273 - 1278, XP008062829
- GOTO T. ET AL.: 'Paternal X-chromosome inactivation in human trophoblastic cells' MOLECULAR HUMAN REPRODUCTION vol. 3, no. 1, 1997, pages 77 - 80, XP008124794
- ZHOU R.-N. AND HU Z.-M.: 'The Development of Chromosome Microdissection and Microcloning Technique and its Applications in Genomic Research' CURRENT GENOMICS vol. 8, no. 1, 2007, pages 67 - 72, XP008124842
- RUSSO S. ET AL.: 'Molecular and genomic characterisation of cryptic chromosomal alterations leading to paternal duplication of the 11p15.5 beckwith-Wiedemann region' JOURNAL OF MEDICAL GENETICS vol. 43, no. 8, 2006, pages E39(1) - E39(9)
- EL-MAARRI O. ET AL.: 'A rapid, quantitative, non-radioactive bisulfite-SnuPE-IP RP HPLC assay for methylation analysis at specific CpG sites' NUCLEIC ACIDS RESEARCH vol. 30, no. 6, 2002, pages E25(1) - E25(4), XP002216942
- KELSEY G. ET AL.: 'Identification of Imprinted Loci by Methylation-Sensitive Representation Difference Analysis: Applications to Mouse Distal Chromosome 2' GENOMICS vol. 62, no. 2, 1999, pages 129 - 138, XP004444714

## Description

### FIELD OF THE INVENTION

The present invention relates to the field of genetics, and more specifically epigenetics. In particular, the invention relates to methods for investigating epigenetic characteristics of the haploid state of an organism.

### BACKGROUND TO THE INVENTION

Epigenetic inheritance is the transmission of information from a cell or multicellular organism to descendants without that information being encoded in the nucleotide sequence of the gene. One type of epigenetic inheritance is DNA methylation, which has been demonstrated to be involved in a number of human diseases. For example, changes in DNA methylation profiles are common in disorders such as cancer, Beckwith-Wiedemann, Prader-Willi and Angelman syndromes. DNA methylation is also known to be involved in modulating gene expression in the course of human development. It is thought that heavy methylation of promoter regions is important in down regulation of transcription, thereby providing a "switch" for gene expression.

DNA methylation is an epigenetic modification that typically occurs at CpG sites (that is, where a cytosine is directly followed by a guanine in the DNA sequence); the methylation results in the conversion of the cytosine to 5-methylcytosine. The formation of Me-CpG is catalyzed by the enzyme DNA methyltransferase. CpG sites are uncommon in vertebrate genomes but are often found at higher density near vertebrate gene promoters where they are collectively referred to as CpG islands. The methylation state of these CpG sites can have a major impact on gene activity and/or expression.

The pattern of methylation has recently become an important topic for research. For instance, both DNA hypomethylation (a loss of methylation) and DNA hypermethylation (an increase in methylation) have been linked to studies examining genes that are differentially methylated between normal and cancerous tissue. The cancer-related genes that have been linked to altered methylation include those involved in cell cycle regulation, DNA repair, RAS signaling and invasion. Studies have found that in normal tissue, methylation of a gene is mainly localised to the coding region, which is CpG poor. In contrast, the promoter region of the gene is unmethylated, despite a high density of CpG islands in the region.

The degree of methylation may also be important in gene regulation. In cancer, epigenetically mediated gene silencing occurs gradually. It begins with a subtle decrease in transcription, fostering a decrease in protection of the CpG island from the spread of flanking heterochromatin and methylation into the island. This loss results in gradual increases of individual CpG sites, which vary between copies of the same gene in different cells.

Another type of epigenetic inheritance is that arising from the effect of DNA-associated proteins. It is known, for example, that the acetylation status of histones can affect expression of the gene with which they are associated. It is thought that acetylation of certain histones leads to silencing of the gene due to the more dense packaging of DNA in the chromatin structure.

While the prior art has disclosed a link between epigenetic modifications to DNA and associated proteins, and phenotype, the interactions are complex and as yet not fully elucidated. It is an aspect of the present invention to overcome or alleviate a problem of the prior art to provide methods for utilizing patterns of epigenetic modifications to DNA and associated proteins patterns to ascribe phenotypes to organisms than that previously thought possible.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### SUMMARY OF THE INVENTION

In a first aspect the present invention provides a method for obtaining epigenetic information for a biological sample obtained from a human, animal or plant subject, the method including the step of:
analyzing the biological sample, the sample containing a substantially isolated DNA molecule, by:
   determining the presence or absence of two or more methylated bases in the DNA molecule,
   wherein the presence or absence of the methylated bases is capable of modulating expression of the DNA molecule *in vivo*; and
   determining whether any two methylated bases are present in *cis* on the DNA molecule;
   characterised in that the substantially isolated DNA molecule is isolated using a physical method; and wherein the substantially isolated DNA molecule consists of a single chromosome or a chromatid, or a fragment thereof.

The step of analyzing may also include an *in situ* method capable of selectively analyzing DNA. The *in situ* method allows for probing of polyploid material to provide definitive haploid epigenetic information, making it unnecessary to physically separate the maternal and paternal DNA molecules.

In another form of the method the DNA molecule is present in, or obtained, from a diploid cell. While gametes contain haploid information, these cells can be difficult to obtain in the clinic and/or provide incorrect information on epigenetic modifications present in somatic cells.

In another form of the invention where the step of analyzing is performed on DNA, the modification is methylation. The analysis may be implemented using any suitable methodology, however typically the step of analyzing the two or more sites for the presence or absence of methylation comprises a method selected from the group consisting of DNA sequencing using bisulfite treatment, restriction landmark genomic scanning, methylation-sensitive arbitrarily primed PCR, Southern analysis using a methylation-sensitive restriction enzyme, methylation-specific PCR, restriction enzyme digestion of PCR products amplified from bisulfite-converted DNA, and combinations thereof.

The epigenetic information provided by the present method may be capable of providing phenotypic information for the subject such as the presence or absence of a disease, condition, or disorder; or a predisposition to a disease, condition, or disorder.

### DETAILED DESCRIPTION OF THE INVENTION

In a first aspect the present invention provides a method for obtaining epigenetic information for a biological sample obtained from a human, animal or plant subject, the method including the step of:
analyzing the biological sample, the sample containing a substantially isolated DNA molecule, by:
   determining the presence or absence of two or more methylated bases in the DNA molecule,
   wherein the presence or absence of the methylated bases is capable of modulating expression of the DNA molecule *in vivo*; and
   determining whether any two methylated bases are present in *cis* on the DNA molecule;
   characterised in that the substantially isolated DNA molecule is isolated using a physical method; and wherein the substantially isolated DNA molecule consists of a single chromosome or a chromatid, or a fragment thereof.

To the best of the Applicant's knowledge, the invention disclosed herein is the first time that the importance of phase when studying epigenetic inheritance by way of DNA and protein modifications has been appreciated. For the first time, phase-specific information is obtained on epigenetic phenomena such as methylation of DNA and acetylation of DNA associated proteins. The present invention therefore provides the ability to discern whether an epigenetic modification at two sites are present on the same chromosome (i.e. a *cis* relationship). This information is important in inter alia identifying phase-specific epigenetic effects in individuals and populations.

The field of epigenetics is relatively new in the art of genetics, and refers to the study of changes in genome function that do not rely on the specific nucleotide sequence within the DNA of an organism. Epigenetics includes the study of effects that are inherited from one cell generation to the next whether these occur in embryonic morphogenesis, regeneration, normal turnover of cells, tumors, cell culture, or the replication of single celled organisms. Specific epigenetic processes of interest include paramutation, bookmarking, gene silencing, X chromosome inactivation, position effect, reprogramming, transvection, imprinting, maternal effects, the progress of carcinogenesis, the effects of many teratogens, and the regulation of histone modifications and heterochromatin.

In another form of the method the presence or absence of the modifications is capable of modulating expression of the DNA molecule *in vivo.* As appreciated by the skilled person, the expression of genes is controlled at least in part by epigenetic modifications such as DNA methylation. DNA methylation is one epigenetic modification of DNA that is proposed to be universal in eukaryotes. In humans, approximately 1 % of DNA bases undergo DNA methylation. In adult somatic tissues, DNA methylation typically occurs in a CpG dinucleotide context; non-CpG methylation is prevalent in embryonic stem cells. In plants, cytosines are methylated both symmetrically (CpG or CpNpG) and asymmetrically (CpNpNp), where N can be any nucleotide.

In one embodiment, the analysing includes determining methylation of a dinucleotide CpG.

In mammals, between 60-70% of all CpGs are methylated. Unmethylated CpGs are grouped in clusters called "CpG islands" that are present in the 5' regulatory regions of many genes. In many disease processes such as cancer, gene promoter CpG islands acquire abnormal hypermethylation, which results in heritable transcriptional silencing. Reinforcement of the transcriptionally silent state is mediated by proteins that can bind methylated CpGs. These proteins, which are called methyl-CpG binding proteins, recruit histone deacetylases and other chromatin remodelling proteins that can modify histones, thereby forming compact, inactive chromatin termed heterochromatin. This link between DNA methylation and protein via alteration to chromatin structure is related to the development of various phenotypes. For example, loss of Methyl-CpG-binding Protein 2 (MeCP2) has been implicated in Rett syndrome and Methyl-CpG binding domain protein 2 (MBD2) mediates the transcriptional silencing of hypermethylated genes in cancer.

As described above, proteins associated with DNA may be involved in the modulation of gene expression. For example, the physical structure of the DNA, as it exists compacted into chromatin, can affect the ability of transcriptional regulatory proteins (termed transcription factors) and RNA polymerases to find access to specific genes and to activate transcription from them.

Chromatin is a term designating the structure in which DNA exists within cells. The structure of chromatin is determined and stabilized through the interaction of the DNA with DNA-binding proteins. There are 2 classes of DNA-binding proteins. The histones are the major class of DNA-binding proteins involved in maintaining the compacted structure of chromatin. There are 5 different histone proteins identified as H1 , H2A, H2B, H3 and H4.

The other class of DNA-binding proteins is a diverse group of proteins simply referred to as non-histone proteins. This class of proteins includes the various transcription factors, polymerases, hormone receptors and other nuclear enzymes. In any given cell there are greater than 1000 different types of nonhistone proteins bound to the DNA.

The binding of DNA by the histones generates a structure called the nucleosome. The nucleosome core contains an octamer protein structure consisting of 2 subunits each of H2A, H2B, H3 and H4. Histone H1 occupies the internucleosomal DNA and is identified as the linker histone. The nucleosome core contains approximately 150 bp of DNA. The linker DNA between each nucleosome can vary from 20 to more than 200 bp. These nucleosomal core structures would appear as beads on a string if the DNA were pulled into a linear structure.

The nucleosome cores themselves coil into a solenoid shape which itself coils to further compact the DNA. These final coils are compacted further into the characteristic chromatin seen in a karyotyping spread. The protein-DNA structure of chromatin is stabilized by attachment to a non-histone protein scaffold called the nuclear matrix.

Histone post-translational modifications (PTMs) associate with positive and negative transcriptional states. A typical model for the role of these PTMs is that in response to cytoplasmic signalling to transcription factors, positive-acting PTMs are established across promoters and open reading frames by DNA- bound activators and RNA polymerase. Negative-acting marks are made across genes during repression by DNA-bound repressor recruitment across heterochromatic regions of the genome. Both sets of modifications alter the nucleosome surfaces which then recruit regulatory protein complexes.

Histone PTMs include, but are not limited to acetylation of histone 3 (H3), histone 4 (H4), histone 2A (H2A), histone 2B (H2B); phosphorylation of H3, H2A and H2B; arginine methylation of H3 and H4; lysine methylation of H3 and H4; lysine ubiquitylation of H2A and H2B; lysine Sumoylation of H2A and H2B; and proline isomerisation in H3; ADP-ribosylation deimination (conversion of arginine to citrulline). The variant histones H2AX, H3.1 , H3.3 and Hzt1 are also modified by PTMs.

This repertoire of histone PTMs, known as the "histone code", serve as binding surfaces for the association of effector proteins containing specific interacting domains. For instance, acteylation is recognised by bromodomains, methylation is recognised by chromo-like domains of the Royal family (chromo, tudor, MBT) and non-related PHD domains, and phosphorylation is recognised by a domain within 14-3-3 proteins.

The histone acetyltransferases (HATs) of interest include the GNAT [GCN5 (general control of amino-acid synthesis 5)-related acetyltransferase] family, the CBP/P300 (CREB-binding protein) family, and the MYST (MOZ, YBF2/SAS3, SAS2, TIP60 protein) family. These HATs, and the transcription factor and nuclear-hormone related histone acetyltransferases, also include GCN5, PCAF (P300/CREB-binding protein-associated factor), GCN5L (general control of amino-acid synthesis 5-like 2), ELP3, P300 (e1a-binding protein p300), CBP (CREB-binding protein), TIP60, MOF/MYST1, MOZ (monocytic leukaemia zinc finger protein)/MYST3, MORF (MOZ-related factor)/MYST4, HB01 (histone acetyltransferase binding to ORC)/MYST2, ATF2, TAF1 (TATA box-associated factor 1), GTF3C4: general transcription factor 3c, polypeptide 4), ACTR: activin receptor, SRC-1 (steroid receptor coactivator 1)/NCOA1/2 (nuclear receptor coactivator 1), ACTR (activin receptor), SRC-1 (steroid receptor coactivator 1), CDYL and HAT1 (histone aceayl transferase 1).

The histone deacetylases (HDACs) of interest include class I and class II HDACs (such as HDACs 1 through 8 and HDAC10), and the class III NAD-dependent enzymes of the Sir family (Such as SirT2).

The histone methyl transferases (HMTs) of interest include both type I and type II arginine HMTs (such as PRMT1, PRMT4, PRMT5 and PRMT7) and lysine HMTs. The lysine HMTs of interest include MLL (Mixed Lineage Leukaemia)-1 through 5, the Set 1 family which have homology to the yeast Set1 protein (including SET1A and SET1 B), SET2, SYMD2, Pr-SET 7/8, CLL8, NSD1, DOT1, SUV42H1/2, EZH2, RIZ1, SUV39H1/2, EuHMTase, GLP, ESET, SETDB1, and G9a.

The arginine and lysine demethylases of interest include protein arginine methylatransferases (PMRT4, PRAT5, and CARM1), LSD1, JHDM1a, JHDM1b, JHDM2a, JHDM2b, JMJD2A/JHDM3A, JMJD2B, JMJD2C, and JMJD2D.

Enzymes mediating other histone PTMs such as deimination, phosphorylation, sumoylation and ubiquitylation that are of interest are PAD14, Bmi/Ring1A, RNF20/RNF40 and UbcH6), mono-ADP-ribosyltransferases and poly-ADP-ribose polymerases, proline isomerases and kinases such as haspin, MSK1/2, CKII and Mst1. Of further interest are proteins binding methylated DNA, such as the methyl-CpG binding domain (MBD) proteins, including MeCP2, MBD1-4, and MBD3L-1, and MBD3L-2.

In addition, since RNAi is involved in heterochromatin formation in insects, plants and fungi, and recent work suggests these mechanisms are present in vertebrates, the present invention includes RNAi-mediated chromatin modifications.

The skilled person will be familiar with methods for isolating DNA associated proteins. Overall DNA 5-methylcytosine content or histone PTMs may be examined using high-performance capillary electrophoresis, high performance liquid chromatography or mass spectrometry. Epigenetic analyses such as restriction landmark genomic scanning may be used to examine haploid genetic material, however approaches utilising amplification (usually by polymerase chain reaction) of target sequences, such as amplification of intermethylated sites (AIMS) or differential methylation hybridization (DMH) are of particular interest. Additionally those that utilise amplification of a signal for detection, such as the detection events of P-LISA (discussed below) facilitating rolling-circle amplification of a hybridisation target.

In order to miniaturise methylation analysis of single chromosomes, it is possible that techniques such as proximity-ligation in situ assay (P-LISA) which are capable of detecting zeptomole amounts (40 x 10⁻²¹ mol) of protein (Fredriksson S et al. (2002) Protein detection using proximity-dependent DNA ligation assays. Nat Biotechnol. 20(5):473-7), may be used. This approach utilises antibodies to the proteins of interest (in one example antibodies against methylated proteins) that, when brought into proximity by binding their target proteins, allow hybridisation of conjugated oligonucleotides which serve as a template for rolling circle amplification upon enzymatic ligation. Since two independent recognition events are required for detection of target proteins, detection is highly specific and capable of detecting individual protein complexes (Soderberg O (2006) Direct observation of individual endogenous protein complexes in situ by proximity ligation. Nat Methods. 2006 3(12):995-1000). This approach has recently been adapted to study protein-DNA interactions, and has been adapted to allow in situ analysis of DNA-protein interactions for localized detection (Gustafsdottir SM et al. (2007) In vitro analysis of DNA-protein interactions by proximity ligation. Proc Natl Acad Sci U S A. 104(9):3067-72).

Chromatin immunoprecipitation coupled to gene array technology (CHIP on chip). In this approach, an antibody to the post-translationally modified DNA-binding protein of interest is used to immunoprecipitate the both the modified protein and its DNA target. In brief, crosslinking is used to fix DNA-binding proteins to DNA, and following fragmentation of DNA, immunoprecipitation is used to purify the protein-DNA fragments with specificity determined by the antibody used. Following hydrolysis to reverse the cross-linking, amplification and labeling of DNA is performed, and the DNA is subsequently hybridized to a microarray and analysed to identify the regions of bound by the DNA-binding protein. Alternately, genes of interest may be examined by PCR using gene specific oligonucleotides (known as single-gene ChIP).

Differential methylation hybridization may also be used to identify methylated DNA. In this approach, specialized DNA microarrays comprising cloned CpG islands are utilised. In the case of the present invention, the DNA samples to be compared - two haploid DNA samples (chromosomes, chromatids etc.) - are each digested with a methylation-sensitive restriction enzyme, and polymerase chain reaction amplicons derived from each sample are then hybridized to the CpG island array. Array elements with a stronger hybridization signal in either sample represent differentially-hypermethylated CpG islands, protected from methylation-sensitive restriction enzyme cleavage, and therefore amplified by PCR in the sample.

Further advances in epigenetic analysis allow examination of epigenetic marks, such as DNA methylation, using diminishing amounts of DNA. For the analysis of DNA methylation, methylated-CpG island recovery assay (MIRA), based on the high affinity of the MBD2/MBD3L1 complex for methylated DNA, has been used to purify methylated DNA. In brief, sonicated DNA is incubated with a matrix containing glutathione-S-transferase (GST)-MBD2b in the presence of MBD3L-1, a binding partner of MBD2 that increases the affinity of MBD2 for methylated DNA. Specifically bound DNA is eluted from the matrix and gene-specific PCR reactions are performed to detect CpG island methylation. Alternately, the bound DNA can be examined using microarray analysis. This technique can already detect methylation using 1 ng of DNA. GST fusions of other proteins involved in epigenesis may also be utilised by such 'pull-down' approaches, or fusions involving biotin to increase affinity for pull-down matricies. Advances in quantitative methylation analysis such as MethyLight and Pryosequencing, when coupled with bisulphite sequencing, allow detection of ever smaller amounts of methylated DNA, with one approach, HeavyMethyl, allowing detection of 30 pg of methylated DNA.

Furthermore, recent advances in top-down proteomics (the analysis of intact proteins rather than first digesting them to peptides) may allow examination the intact modification patterns of different histones in a given nucleosome.

While there have been many studies on the mechanisms of epigenesis, the prior art has not adequately appreciated the confounding effects of concurrently considering he epigenetic modification of a maternally-derived DNA molecule or protein at the same time as a paternally-derived DNA molecule or protein. This has occurred because the methods of the prior art analyze epigenetic modifications using diploid material.

Applicant proposes herein that a definitive characterization of the epigenetic state of a subject can only be provided by separately analyzing epigenetic modification of DNA and associated proteins in the haploid state. For example, where methylation is the epigenetic modification, use of haploid material resolves phase-specific effects, for recognition of heterozygous phenomena and provides for analysis of quantitative variation over the multiple CpG sites comprising a (unitarily functional) 'island', even for variation at a single CpG site. This definitive characterization leads to more accurate phenotypic information, such as the presence and/or predisposition of an individual to certain diseases or the ability of an individual to respond to certain therapeutic molecules.

Considering a more specific example, DNA methylation is an epigenetic feature that is thought to be involved in the pathogenesis of cancer. The following table describes cancer-related genes thought to be methylation sensitive.

| **Gene** | **Map** | **Methylated** | **Notes** |
|---|---|---|---|
| 14-3-3 Sigma | 1 p | Breast and gastric cancers | |
| ABL1 (P1) | 9q34.1 | 50-100% CML, Some ALL | Only methylated when part of the bcr-abl translocation. |
| ABO | 9q34 | cell lines | |
| APC | 5q21 | Colon, gastric and esophageal cancer | One promoter only. Correlation with expression not established. Type A |
| AR (Androgen Receptor) | Xq11-12 | Prostate Cancer Cell Lines, Colon ACFs | |
| BLT1 (Leukotriene B4 Receptor) | | Various cell lines | |
| BRCA1 | 17q21 | 10-20% Breast cancer, some ovarian | Cause of transcriptional silencing in these cells |
| CALCA (Calcitonin) | 11p15 | 25-75% Colon, lung, hematopoic neoplasms. | One of the first promoter-CpG islands demonstarted to be hypermethylated in cancer. |
| CASP8 (CASPASE 8) | 2q33-34 | Neuroblastoma | Corralates with MycN amplification |
| Caveolin 1 | 7q31.1 | Breast cancer cell lines | |
| CD44 | 11pter-p13 | Prostate cancer | |
| CFTR | 7q31.2 | Cell Lines | No primary tumors reported |
| COX2 | 1q25.2-q25.3 | Colon, Breast and prostate cell lines. 15% of primary colon cancers | Correlates with expression when completely methylated. |
| CSPG2 (Versican) | 5q12-14 | AGING Colon. 70% colon | Secreted proteoglycan, regulated by Rb. |
| CX26 (Connexin 26) | 13q11-q12 | Breast cancer cell lines | |
| Cyclin A1 | 13q12.3-q13 | Various cell lines | |
| DBCCR1 | 9q32-33 | 50% Bladder cancer | Slight methylation in normal bladder |
| ECAD (E-cadherin) | 16q22.1 | 20-70% Breast, Gastric, Thyroid, SCC, Leukemias and Liver ca. | Methylation is often heterogeneous and not always correlated with silencing. Also present in some normal stomach and liver samples |
| Endothelin Receptor B | 13q22 | 60-70% prostate cancer | |
| EPHA3 | 3p11.2 | Leukemias | |
| EPO (Erythropoietin) | 7q21 | HeLa Cells | Normal and primary tumors |
| ER (Estrogen Receptor) | 6q25.1 | AGING colon, liver, heart muscle, AoSMC (cultured), brain, AoEC. 100% Colon cancer 20-30% ER-breast cancer 60-70% AML/ALL 20-50% CML-BC 20% Lung (NSCLC) 60% GBM | Upstream promoter not in CpG island. Nevertheless, there is a good correlation with loss of expression. |
| FHIT | 3p14.2 | 10-20% Esophageal SCC | |
| GPC3 (Glypican 3) | Xq26 | Mesothelioma and Ovarian cancer cell lines | |
| GST-pi | 11q13 | 80-100% Prostate, Liver. 30-60% Colon, Breast, Kidney. | DNA repair/detoxification enzyme. |
| H19 | 11 p15.5 | 20-50% Wilm's tumors | Imprinted gene. Hypermethylation is associated with apparent loss of imprinting of the IGF2 gene in Wilm's tumor, but not others. |
| H-Cadherin (CDH13) | 16q24.1-24.2 | 45% Lung Cancer, some ovarian cancer | |
| HIC1 | 17p13.3 | Prostate, Breast and Brain,. 80-100% Colon cancer, Prostate, Breast, GBM. 20-50% Lung, Kidney, Liquid tumors. | Candidate tumor-suppressor gene. First gene cloned based on finding a CpG island hypermethylated in cancer. |
| hMLH1 | 2p22 | 10-20% colon, endometrial and gastric cancers. 0% lung, breast, GBM, liquid tumors etc. | Almost always associated with microsatellite instability and, in celllines, mismatch repair deficiency. |
| HOXA5 | 7p15-p14.2 | Breast cancer | |
| IGF2 (Insulin-Like Growth Factor II) | 11 p15.5 | AGING colon 100% Colon cancer 50% AML | IGF2 has a large CpG island that contains the imprinted P2-4 promoters |
| IGFBP7 | 4q12 | Murine SV40 T/t antigen-induced hepatocarcinogenesis | Normal and primary tumors |
| IRF7 | 11 | Various cell lines | |
| LKB1 | 19p13.3 | A few colon, testicular and breast (medullary) primary tumors | |
| LRP-2 (Megalin) | 2q24-31 | Various cell lines | |
| MDGI (Mammary-derived qrowth inhibitor) | 1p35-33 | 50-70% Breast cancers | |
| MDR1 | 7q21.1 | Drug sensitive leukemia cell lines. | Primary tumors |
| MDR3 (PGY3) | 7q21.1 | Various cell lines | |
| MGMT (06 methyl guanine methyl transferase) | 10q26 | 25-50% Brain, colon, lung, breast, NHL etc. | Associated with the MER-phenotype |
| MT1a (metallothionein 1) | 16q13 | Rat hepatoma | Normal and primary tumors |
| MUC2 | 11 p15.5 | Colon cancer cell line | Primary tumors |
| MYOD1 | 11 p15.4 | AGING Colon. 100% colon, 30% breast, Also bladder, lung, liquid tumors. | |
| N33 | 8p22 | AGING Colon. 60-80% colon, prostate, brain. | Oligo-saccharyl-transferase |
| NEP (Neutral Endopeptidase 24.1) / CALLA | 3q21-27 | Prostate cancer (~10%) | |
| NF-L (light-neurofilament-encoding qene) | 8p21 | Rat Glioma cell line | |
| NIS (sodium-iodide symporter gene) | 19p13.2-p12 | Thyroid cancer cell lines | Heterogeneous methylation in primary tumors |
| P14/ARF | 9p21 | Colon cancer cell lines (infrequent) | Less frequent than P16 methylation, but usually associated with P16 methylation. |
| P15 (CDKN2B) | 9p21 | 80% AML/ALL 2-20% GBM 0% Colon/Lung/Breast | P15 is physically close to P16, but simultaneous methylation of both qenes is rare. |
| P16 (CDKN2A) | 9p21 | 20-30% Lung (NSCLC) 25-35% Colon 5-25% Lymphomas (depending on stage) 0-5% Bladder. Many others (esophagus, stomach etc.) | Methylation is as frequent as deletions, and more frequent than mutations. |
| P27KIP1 | 12p13 | Rodent pituitary cancer cell lines | No primary tumors reported |
| p57 KIP2 | 11 p15.5 | Gastric cancer cell lines | |
| PAX6 | 11p13 | Colon cancer cell lines and 70% of primary tumors | |
| PgR (Progesterone Receptor) | 11q22 | 10-20% Breast cancer | Effect on transcription |
| RAR-Beta2 | 3p24 | Colon, Breast, Lung Cancer | |
| RASSF1 | 3p21.3 | Lung cancer | One promoter only |
| RB1 (Retinoblastoma) | 13q14 | 10-20% Retinoblastomas 0% Lung/Leukemia/Colon Some pituitary adenomas | |
| TERT | 5p15.33 | Heterogeneous methylation in many cell lines | |
| TESTIN | 7q31.2 | Hematopoietic malignancies | One promoter only |
| TGFBRI | 9q33-q34 | Gastric cancer cell lines and primary tumors (10%) | |
| THBS1 (Thrombospondin-1) | 15q15 | 5-10% Colon Cancer 30-40% GBM 20-30% AML 0% Endometrial/Breast | Angiogenesis inhibitor, regulated by P53 and Rb in some systems. |
| TIMP3 | 22q12.1-13.2 | Human brain (10-50%) and kidney (20%) cancers, Mouse model | |
| TLS3 (T-Plastin) | X | Leukemia cell lines | |
| Urokinase (uPA) | 10q24 | Breast cancer cell lines | |
| VHL (Von-Hippell Lindau) | 3p25-25 | 10-20% Renal Cell cancers 0% Common solid and liquid tumors | Same tumor selectivity as mutations |
| WT1 | 11p13 | 90% Breast cancers, 20-50% colon, 5-10% Wilm's | Correlation with expression |
| ZO2 (Zona Occludens 2) | | Pancreatic cancer cell lines | |

As will be noted from the above Table, the prior art has appreciated that methylation is important in the pathogenesis of cancer, however only diploid material has been used to date. Applicant proposes that methods of the prior art are susceptible to providing less than accurate information. For example, upon profiling a given promoter in a tumor sample, a moderate degree of methylation may be noted. In real terms the degree of methylation measured is an average of methylation for the maternally derived promoter sequence and the paternally derived promoter sequence, since the tumor is of course diploid. While this averaged result may be true in some cases (because both maternal and paternally derived promoters are methylated to the same extent), this will not be universally true. For example, the maternal promoter sequence could be completely unmethylated, and the paternal sequence may be heavily methylated. If the maternally-derived sequence is dominant over the paternal sequence in terms of promoter activity, then the importance of analyzing the maternal and paternal sequences separately becomes apparent.

It will be understood from the foregoing that the presence or absence of an epigenetic modification is analyzed with reference to the haploid state of the cell. Thus, the presence or absence of methylation at a given DNA site under consideration may determine where maternally derived epigenetic feature is substantially separated from the counterpart paternally derived epigenetic features. In this way, the influence of maternally derived epigenetic features can be ascertained in the absence of paternally derived epigenetic features, and *vice versa*. Applicant proposes that a more accurate methylation map is gained by removing the potentially inaccurate (or at least less than definitive) results provided where diploid material is analyzed.

The skilled artisan will appreciate that the present invention is distinguished from the natural process of genomic imprinting whereby the level of expression of some genes depends on whether or not they are inherited from the maternal or paternal genome. For example, insulin-like growth factor-2 (IGF2) is a gene whose expression is required for normal fetal development and growth. Expression of IGF2 occurs exclusively from the paternal copy of the gene. Imprinted genes are "marked" by their state of methylation. In the case of IGF2 an element in the paternal locus, called an insulator element, is methylated blocking its function. The function of the un-methylated insulator is to bind a protein that when bound blocks activation of IGF2 expression. When methylated, the protein cannot bind the insulator thus allowing a distant enhancer element to drive expression of the IGF2 gene. In the maternal genome, the insulator is not methylated, thus protein binds to it blocking the action of the distant enhancer element.

By "definitive", it is meant that no estimate or inference or determination of likelihood or probability is involved in assigning a certain epigenetic modification pattern to a certain phenotype. Methods of analyzing epigenetic modifications described in the prior art are confounded by the presence of paternal modifications in combination with maternal modifications. Thus, while certain algorithms may be used to deduce or infer a likely assignment between a given methylation pattern and phenotype for example, where non-haploid material is analyzed these assignments will necessarily be flawed. It is proposed that the confusion often noted in assigning a methylation pattern to a phenotype results at least in part by the confounding influence of diploid material.

According to the present method, a DNA is obtained from a biological sample of the human, animal or plant subject. The biological sample may be any material that contains DNA including but not limited to whole blood, serum, a blood cell, a skin cell, saliva, urine, hair, nails, tears, nails, and the like.

Where the step of analyzing includes the substantial isolation of DNA, the skilled artisan may use any suitable method known to him or her. In the method the step of substantially isolating the DNA is by physical means. The advantage provided by physical means over non-physical means (such as selective probing of diploid material) is that problems associated with discerning maternally derived material from paternally derived material are avoided. For example, where selective probes are used, it could be that cross- hybridization is problematic leading to uncertain results. While hybridisation conditions can be varied to limit cross-hybridisation, this requires further experimentation to be performed.

In one form of the invention, the physical method to provide haploid DNA containing exclusively paternally-derived DNA or maternally-derived DNA is chromosome microdissection. The substantially isolated DNA molecule consists of a single chromosome, a chromatid or a fragment of a chromatid.

Conveniently, cuts in the chromosome may be made distal to the centromere to separate the p and q arms, each being a haploid DNA molecule. The skilled person is enabled to identify the physical region of interest in a chromosome and adopt an appropriate method to isolate haploid DNA from a diploid, tripolid, tetraploid, or any other sample having a higher level of ploidy.

The skilled person is familiar with platforms and tools used for micromanipulation. Although technically exacting, microdissection is routinely achievable. Equipment requirements consist of a microscope (either upright or inverted) fitted with a micromanipulator and a rotating stage, and a pipette puller (to produce microneedles). Vibration isolation for the microscope is recommended. Although a special clean room is not required, microdissected chromosome fragments contain only femptogram quantities of DNA, and contamination with extraneous DNA must be controlled.

In one form of the method, a non-contact method for isolating a haploid DNA molecule may be used. An example of this approach is the use of a laser microbeam. Laser microbeam microdissection may involve use of a pulsed ultraviolet laser of high beam quality interfaced with a microscope. Laser beam microdissection may be performed using, for example, a commercially available P.A.L.M.® Robot Microbeam (P.A.L.M. GmbH Bernried, Germany). The light laser is preferably of a wavelength that does not damage or destroy the genome segment, such as 337nm which is remote from the absorption maximum of nucleic acids such as DNA.

Another useful system for laser microdissection of chromosomes is the Leica Laser Microdissection Microscope. The system uses a DMLA upright microscope including motorized nosepiece, motorized stage, the xyz-control element and all other advantages of the new DMLA microscope. The laser used is a UV laser of 337 nm wavelength. The movement during cutting is done by the optics, while the stage remains stationary. The region of interest can be marked on the monitor and is cut out by PC control. The sample falls down into PCR tubes without extra forces. The result of the cutting can be easily checked by an automated inspection mode.

Isolation of a haploid DNA molecule may be achieved by, for example, microdissection using laser catapulting of a chromosome segment using a PALM laser. In this case, the non-contact process involves laser ablation around the targeted chromosome element, followed by laser force catapulting of the defined element onto a tube cap, such as a microcentrifuge tube cap, for subsequent analysis of single arm DNA.

The resultant isolated haploid DNA molecule may be recovered using laser pressure catapulting. Laser pressure catapulting may be achieved by focussing a laser microbeam under, for example a haploid genome segment or segments of interest, and generating a force as a result of the high photon density that develops and causes the required haploid material to be catapulted from the non-required genome segment. The sample travels on the top of a photonic wave and is catapulted into a collection tube. Suitable collection tubes will be known to those of skill in the art and include tubes such as a common polymerase chain reaction (PCR) reaction tube or a microcentrifuge tube.

The DNA may be substantially isolated by preparative flow cytometry using probes capable of discriminating between maternal and paternal DNA. Another method is by the use of radiation hybrids, where the development of diploid material involves human chromosomes as only one of each chromosome pair. Another strategy is the use of "conversion technology", as developed by GMP Technologies Inc. GMP Conversion Technology(R) utilizes a process to separate paired chromosomes into single chromosomes. When separated, alleles may be analyzed individually using genetic probes that identify gene sequences. This technology is applicable to a gene, a chromosome, or to the entire human genome.

In another form of the method the contaminant genetic material is inactivated or ablated such that it no longer performs the function of contaminant genetic material. For example, where it is desired to isolate a maternally-derived DNA, the paternal contribution may be inactivated or ablated. This may be achieved by destroying a homologous chromosome using a carefully directed laser beam for example.

Another method for selectively analysing a DNA molecule is to selectively amplify a haploid sequence using PCR such that the number of copies of haploid DNA is in vast excess over that of the contaminant DNA. The mixture of DNA molecules could then be partially digested with a nuclease such that substantially all contaminant DNA is digested, and a low level of haploid DNA remains.

The possibility also exists for selectively amplifying the haploid DNA by long PCR using primers incorporating a tag, and separating out the copies using the tag.

Once the DNA is substantially isolated, analysis is undertaken to determine the presence or absence of an epigenetic modification.

Where the epigenetic modification is the methylation of DNA, methylation may be detected by analyzing the number 5 carbon of the cytosine pyrimidine ring for the presence or absence of a methyl group.

The method may be implemented using any suitable methodology, however typically the step of analyzing the two or more sites for the presence or absence of methylation comprises a method selected from the group consisting of DNA sequencing using bisulfite treatment, restriction landmark genomic scanning, methylation-sensitive arbitrarily primed PCR, Southern analysis using a methylation-sensitive restriction enzyme, methylation-specific PCR, restriction enzyme digestion of PCR products amplified from bisulfite-converted DNA, and combinations thereof.

Bisulfite sequencing involves reacting single-stranded DNA with sodium bisulfite, which selectively deaminates cytosine to uracil but does not react with methylcytosine. The modified DNA sequence produced in the bisulfite reaction is amplified by PCR, and then the amplified DNA is ligated into a plasmid vector for cloning and sequencing. When the DNA is sequenced, only the intact methylated cytosine residues are amplified as cytosine.

Bisulfite sequencing can be performed using DNA isolated from fewer than 100 cells, which is one of the major advantages of this tool, because tumor specimens are typically very small. Other benefits of bisulfite sequencing include its ability to analyze long stretches of the genome to determine very clear patterns of methylation in the DNA, and it yields a quantitative positive display of 5-methylcytosine residues.

MSP is a very rapid and sensitive technique for methylation screening. MSP is performed using sodium bisulfite to modify the DNA and convert unmethylated cytosines to uracil. Subsequent amplification is performed with primers specific for the methylated versus unmethylated DNA, and the analysis is performed with simple gel electrophoresis. MethyLight is the next generation of the MSP assay. The work up of the sample and the premise of the assays are identical. The MethyLight approach is an advance: While maintaining the exquisite sensitivity provided by standard MSP, the assay is made more quantitative, and less labor intensive through the incorporation of a real time "TaqMan" PCR format.

The most critical parameter affecting the specificity of methylation-specific PCR is determined by primer design. In practice, it is often preferred to deal with only one strand, most commonly the sense strand. In one form of the method, primers are designed to amplify a region that is 20-30 bp in length, and should incorporate enough cytosines in the original sequence to assure that unmodified DNA will not serve as a template for the primers. In addition, the number and position of cytosines within the CpG dinucleotide determines the specificity of the primers for methylated or unmethylated templates. Typically, 1-3 CpG sites are included in each primer, and concentrated in the 3' region of each primer. This provides optimal specificity and minimizes false positives due to mispriming. To facilitate simultaneous analysis of the U and M reactions of a given gene in the same thermocycler, we adjust the length of the primers to give nearly equal melting/annealing temperatures. This usually results in the U product being a few base pairs larger than the M product, which provides a convenient way to recognize each lane after electrophoresis.

Since methylation-specific PCR utilizes specific primer recognition to discriminate between methylated and unmethylated sites, it is preferred that stringent conditions are maintained for amplification. This means that annealing temperatures should be close to the maximum temperature which allows annealing and subsequent amplification. In practice, new primers are typically tested with an initial annealing temperature 5-8 degrees below the calculated melting temperature. Non-specificity can be remedied by slight increases in annealing temp, while lack or weak PCR products may be improved by a drop in temperature of 1 -3 degrees Celsius. As with all PCR protocols, care should be taken to ensure that the template DNAs and reagents do not become contaminated with exogenous DNAs or PCR products.

MSP utilizes the sequence differences between methylated alleles and unmethylated alleles which occur after sodium bisulfite treatment. The frequency of CpG sites in CpG facilitate this sequence difference. Primers for a given locus are designed which distinguish methylated from unmethylated DNA in bisulfite-modified DNA. Since the distinction is part of the PCR amplification, extraordinary sensitivity, typically to the detection of 0.1 % of alleles can be achieved, while maintaining specificity. Results are obtained immediately following PCR amplification and gel electrophoresis, without the need for further restriction or sequencing analysis. MSP also allows the analysis of very small samples, including paraffin-embedded and microdissected samples.

In one embodiment of the method the step of analyzing the two or more sites for the presence or absence of methylation analysis comprises: (a) reacting the DNA with sodium bisulfite to convert unmethylated cytosine residues to uracil residues while leaving any 5-methylcytosine residues unchanged to create an exposed bisulfite-converted DNA sample having binding sites for primers specific for the bisulfite-converted DNA sample; (b) performing a PCR amplification procedure using top strand or bottom strand specific primers; (c) isolating the PCR amplification products; (d) performing a primer extension reaction using a Ms-SNuPE primer, dNTPs and Taq polymerase, wherein the Ms-SNuPE primer comprises from a 15-mer to a 22-mer length primer sequence that is complementary to the bisulfite-converted DNA sample and terminates immediately 5' of the cytosine residue of the two or more CpG dinucleotide sequences to be assayed; and (e) determining the methylation state of the two or more CpG dinucleotide sequences by determining the identity of the first primer-extended base.

In one embodiment, the dNTPs are labelled, and determining the identity of the first primer-extended base is measured by incorporation of the labelled dNTPs

Methylation analysis may be facilitated by the use of high throughput methods to identify sites of potential methylation. Techniques available for screening include restriction landmark genome scanning (RLGS); gene expression arrays, which may used as a surrogate to see what genes are expressed after exposure to DNA methyltransferase inhibitors like azacitidine.

Highly parallel genome-wide assays are known in the art, with a number disclosed by Fan et al (Nat Rev Genet 2006, 7(8) 632-44). Many such methods are available to the skilled person as contract services, an example being the Golden Gate Methylation Solution" such as that provided by Illumina Inc (San Diego, CA). The Illumina system is capable of analyzing up to 1,536 CpG sites at single-site resolution over hundreds of genes across 96 samples. This system can therefore provide up to 147,456 quantitative DNA methylation measurements per assay.

It is further contemplated that a human CpG island library be obtained, and then arrayed. Database construction begins with crude chromatograms. After duplicates are removed, all 6,800 elements are subjected to Basic Local Alignment Search Tool (BLAST) analysis against the University of California, Santa Cruz, High Throughput Genomic and nr DNAsequence databases and were sequenced. In the CpG island database, each clone is assigned an identification number and its characteristics are listed, including such information as its chromosome location, whether it is a promoter or found in the 5' flanking region, its GC content, and what restriction sites exist in the clone. The latter information is useful for determining the utility of restriction enzyme analysis as well as the actual sequence.

The CGI microarray technique is also contemplated to be useful in the context of the present methods. This technique permits simultaneous assessment of thousands of potential targets of DNA methylation on a single chip. It involves arraying of CpG island clones on glass slides, preparation of target sample amplicons, and hybridization of the amplicons onto the CGI microarrays. As an example, the technique may be performed using tumor derived genomic DNA samples that are obtained using a restriction enzyme (Mse1) able to cut immediately outside of the CpG island. Next, catch linkers bearing PCR primer sequences are added to the Mse1 fragments. The sample is split into a reference portion, which serves as a denominator to determine how well the genome amplification worked, and into a test portion that is digested with McrBC, a methylation sensitive restriction enzyme that only digests DNA if the region is methylated. The two portions are amplified by PCR, and then the DNA is direct labeled with Cy5 red (test) or Cy3 green (reference) fluorescent dyes. DNA fragments not digested by McrBC in the test sample produce Cy5-labeled PCR product while no labeled PCR product is produced if there were methylated fragments digested by McrBC. The labeled test and reference PCR products are mixed and spotted onto the glass slide. The hybridized slides are scanned and the acquired images analyzed to identify methylated signals.

As will be appreciated by the skilled person, the present invention will have many uses in the filed of biology, and particularly in medicine. As discussed *supra*, cancer is considered an epigenetic In fact, epigenetic changes, particularly DNA methylation, are susceptible to change and are excellent candidates to explain how certain environmental factors may increase the risk of cancer. The delicate organization of methylation and chromatin regulates the normal cellular homeostasis of gene expression patterns becomes unrecognizable in the cancer cell. The genome of the transformed cell may simultaneously undergo a global genomic hypomethylation and a dense hypermethylation of the CpG islands associated with gene regulatory regions. These dramatic changes may lead to chromosomal instability, activation of endogenous parasitic sequences, loss of imprinting, illegitimate expression, aneuploidy, and mutations, and may contribute to the transcriptional silencing of tumour suppressor genes. The hypermethylation-associated inactivation may affect many of the pathways in the cellular network, such as DNA repair (hMLH1, BRCA1, MGMT, em leader), the cell cycle (p16(INK4a), p14(ARF), p15(INK4b), and apoptosis (DAPK, APAF-1) The aberrant CpG island methylation can also be used as a biomarker of malignant cells and as a predictor of their behaviour.

In one form of the invention the haploid DNA is present in, or obtained, from a diploid cell. The method may use an autosomal chromosome of a somatic cell. The term "autosomal chromosome" means any chromosome within a normal somatic or germ cell except the sex chromosomes. For example, in humans chromosomes 1 to 22 are autosomal chromosomes. Applicant proposes that avoidance of naturally haploid material (such as that contained in sperm and ova) is advantageous because epigenetic modifications such as methylation are at least partially erased, and are often completely erased. This is because; the pattern of methylation is typically reset during meiosis. Thus, analysis of a sperm cell or ovum will not provide useful information allowing the definition of a phenotype for the subject.

The use of naturally haploid material such as sperm cells or ova is also to be avoided due to problems with obtaining these sex cells in the clinic. Obtaining ova is an invasive procedure for females of any age, and harvesting sperm cells from a pediatric male also requires medical intervention.

The avoidance of sex cells in epigenetic haplotyping has a further advantage when it is considered that during the process of meiosis recombination events may occur such that loci that were formerly linked in *cis,* become associated in *trans.* Thus, analysing an epigenetic haplotype of a gamete will give different (i.e. incorrect) haplotype information to that of haploid DNA obtained from a diploid cell.

In one embodiment of the invention, two or more sites on the same DNA molecule are analyzed for methylation. This form of the method is useful in determining whether the two methylated sites are naturally present in *cis* on the DNA molecule in the cell. This may be achieved by considering only the methylation sites on a single chromosome (either maternally or paternally-derived), for example to demonstrate that the two methylation sites are present in *cis* because they both appear on the same chromosome.

The method may be practiced where the DNA is not physically separated into paternally-and maternally derived components. By using an *in situ* method it will be possible to selectively probe a paternal DNA or a maternal DNA such that it is unnecessary to physically separate the two molecules. In this way, diploid material may be used to provide information on the haploid state of the cell. Methods such as primed *in situ* labelling (PRINS) will be useful in this regard by selectively identifying maternal or paternal DNA thereby allowing the localisation of an epigenetic modification to a maternal or paternal chromosome. The method has been successfully used with primers specific for certain chromosomes, using both metaphase and interphase nuclei (Hindkjaer et al, Methods Mol Biol. 1994; 33:95-107).

It is also contemplated that PNA probes can be utilised for the *in situ* identification of chromosomes. PNA chemistry can be used to fabricate small oligomers. When dye-labeled, these oligomers make excellent probes and offer distinct advantages over conventional nucleic acids. PNA probes are typically very small (12 to 20 mers), and demonstrate both strong signals and very low background. PNA probes can be used in the context of fluorescence *in situ* hybridisation (PNA-FISH) as a powerful technology to explore chromosome structure on the metaphase and interphase chromosome. PNA- FISH is a method where small PNA oligomer probes are used to label chromosomes in a sequence specific manner, allowing identification of an underlying sequence in a particular chromosome. Methods for PNA-FISH are disclosed in Strauss 2002 ("PNA-FISH" in FISH Technology. Rautenstrauss/Liehr Eds. Springer Verlag. Heidelberg).

It will be understood that the methods described herein may find use in any area of biology where the effect of methylation and other protein modifications must be considered in connection with gene expression. These uses are not limited to animals (human or otherwise), and may also be applied to plants.

Phenotypic information obtained from the present methods is selected from the group consisting of: the presence or absence of a disease, condition, or disorder, or a predisposition to a disease, condition, or disorder.

The present invention will now be more fully described by reference to the following nonlimiting examples.

### EXAMPLE 1: METHYLATION SPECIFIC PCR ON HAPLOID DNA

### Tissue Preparation

Metaphase spreads of peripheral blood cells obtained from a human subject are prepared by standard karyotyping methods onto PEN membrane slides. A standard Giemsa stain is prepared and used to identify chromosomes in the preparation.

### Staining Procedure

| Step | Process | Time |
|---|---|---|
| 1. | Phosphate buffer, pH6.8 | 1 minute |
| 2. | 0.025% Trypsin in Phosphate Buffer, pH 6.8 | 2 minutes |
| 3. | Phosphate Buffer, pH 6.8 | 1 minute |
| 4. | Giemsa stain, working solution | 15 minutes |
| 5. | Distilled water | 2-3 dips |

Excess water is shaken off the slide, and the backside dried with a kimwipe and left to air dry.

### Laser Microdissection

The PALM™ Microdissection system is used (P.A.L.M. Microlaser Technologies GmbH, Germany). Metaphase spreads (as prepared above) are used for laser capture using a P.A.L.M microscope under a 100x objective lens. Single metaphase chromosomes spatially separated from their sister chromosome, including single chromosomes are catapulted into the caps of 200ul UltraFlux Flat Cap PCR tubes containing 6µl of 0.1%(v/v) Triton-X-100 using standard P.A.L.M microscope protocols. The catapulted material was transferred to the bottom of the tube by centrifugation for methylation analysis. Both maternally-derived and paternally-derived DNA samples are obtained.

### DNA Extraction

DNA from the microdissected chromosome fragment on the CapSure Macro LCM cap is extracted using PicoPure DNA Extraction Kit (Arcturus Inc, CA) following the kit protocol.

### Detection of methylation:

DNA is modified by sodium bisulfite treatment converting unmethylated, but not methylated, cytosines to uracil. Following removal of bisulfite and completion of the chemical conversion, this modified DNA is used as a template for PCR. Two PCR reactions are performed for each DNA sample, one specific for DNA originally methylated for the gene of interest, and one specific for DNA originally unmethylated. PCR products are separated on 6-8% non-denaturing polyacrylamide gels and the bands are visualized by staining with ethidium bromide. The presence of a band of the appropriate molecular weight indicates the presence of unmethylated, and/or methylated alleles, in the original sample.

To prepare the mixes 10x PCR buffer, NTP's and primers are thawed. The number of samples to be analyzed is determined, including a positive control for both the unmethylated and methylated reactions, and a water control. A master mix for each PCR reaction (methylated and unmethylated) is made. For each 50 µl reaction, the following amounts should be used:

| | |
|---|---|
| 10x PCR buffer: | 5 µL |
| 25mM 4 NTP mix | 2.5 µL |
| Sense primer (300ng/µL) | 1 µL |
| Antisense primer (300ng/µL) | 1 µL |
| Distilled, sterile water | 28.5 µL |

38 µl of this PCR mix is dispensed into separate PCR tubes (0.5 mL tubes or strips) labeled for each sample. The components are well mixed prior to dispensing.

2 µl of bisulfite modified DNA template is added to each tube. An unmethylated and methylated reaction is included for each sample, as are positive controls, as well as a no DNA control.

One to two drops of mineral oil (~25-50 µl) are added to each tube, before placing in thermocycler. The mineral oil completely covers the surface of the reaction mixture to prevent evaporation.

The PCR products are then amplified in the thermal cycler. The PCR is initiated with a five-minute denaturation at 95°C. Taq polymerase is added after initial denaturation: 1.25 units of Taq polymerase, diluted into 10 µl of sterile distilled water. This 10 µl is mixed into the 40 µl through the oil by gently by pipetting up and down. Amplification is continued for 35 cycles with the following parameters:
30 sec 95 °C (denaturation)
30 sec specific for primer (annealing)
30 sec 72 °C (elongation)
Final step: 4 min 72 °C (elongation)
Store at 40 °C until analysis.

The PCR products are analyzed by gel electrophoresis as follows.

A 6-8% non-denaturing polyacrylamide gel is prepared. 1 X TBE provides better buffering capacity and sharper bands for resolving these products. The size of the products typically generated by MSP analysis is in the 80-200 bp range, making acrylamide gels optimal for resolution of size. High-percentage horizontal agarose gels can be used as an alternative.

Reactions from each sample are run together to allow for direct comparison between unmethylated and methylated alleles. Positive and negative controls are included. Vertical gels are run at 10 V/cm for 1 -2 hours. The gel is stained in ethidium bromide, and visualized under UV illumination. A comparison of the methylation status of maternally-derived DNA and paternally-derived DNA for a given DNA region is made.

## Claims

1. A method for obtaining epigenetic information for a biological sample obtained from a human, animal or plant subject, the method including the step of:
analyzing the biological sample, the sample containing a substantially isolated DNA molecule, by:
determining the presence or absence of two or more methylated bases in the DNA molecule, wherein the presence or absence of the methylated bases is capable of modulating expression of the DNA molecule *in vivo*; and
determining whether any two methylated bases are present in *cis* on the DNA molecule;
**characterised in that** the substantially isolated DNA molecule is isolated using a physical method; and wherein the substantially isolated DNA molecule consists of a single chromosome or a chromatid, or a fragment thereof.

2. The method according to claim 1, wherein the single chromosome or the chromatid, or fragment thereof is isolated by laser-mediated dissection.

3. The method according to claim 1 or 2, wherein the DNA molecule is present in, or obtained from, a diploid cell.

4. The method according to any one of claims 1 to 3, wherein the analysing includes determining methylation of a dinucleotide CpG.

5. The method according to either claim 4 or claim 5, wherein the analyzing includes a method selected from the group consisting of DNA sequencing using bisulfite treatment, restriction landmark genomic scanning, methylation-sensitive arbitrarily primed PCR, Southern analysis using a methylation-sensitive restriction enzyme, methylation-specific PCR, restriction enzyme digestion of PCR products amplified from bisulfite-converted DNA, and combinations thereof.

6. The method according to claim 5, wherein where the analyzing includes DNA sequencing using bisulphite treatment, the analyzing includes:
(a) reacting the DNA with sodium bisulfite to convert unmethylated cytosine residues to uracil residues while leaving any 5-methylcytosine residues unchanged to create an exposed bisulfite-converted DNA sample having binding sites for primers specific for the bisulfite-converted DNA sample;
(b) performing a PCR amplification procedure using top strand or bottom strand specific primers;
(c) isolating the PCR amplification products;
(d) performing a primer extension reaction using a Ms-SNuPE primer, dNTPs and Taq polymerase, wherein the Ms-SNuPE primer comprises from a 15-mer to a 22-mer length primer sequence that is complementary to the bisulfite-converted DNA sample and terminates immediately 5' of the cytosine residue of the two or more CpG dinucleotide sequences to be assayed; and
(e) determining the methylation state of the two or more CpG dinucleotide sequences by determining the identity of the first primer-extended base.

7. The method of claim 6, wherein the dNTPs are labelled, and determining the identity of the first primer-extended base is measured by incorporation of the labelled dNTPs.

8. The method according to any one of claims 1 to 7, wherein the epigenetic information is capable of providing phenotypic information for the subject from which the biological sample was obtained.

9. The method according to claim 8, wherein the phenotypic information is selected from the group consisting of: the presence or absence of a disease, condition, or disorder; or a predisposition to a disease, condition, or disorder.

## Patentansprüche

1. Verfahren zum Einholen von epigenetischen Informationen für eine biologische Probe, die von einem Menschen-, Tier- oder Pflanzensubjekt gewonnen wurde, wobei das Verfahren den folgenden Schritt beinhaltet:
Analysieren der biologischen Probe, wobei die Probe ein im Wesentlichen isoliertes DNA-Molekül enthält, durch:
Ermitteln der An- oder Abwesenheit von zwei oder mehr methylierten Basen in dem DNA-Molekül, wobei die An- oder Abwesenheit der methylierten Basen die Expression des DNA-Moleküls *in vivo* modulieren kann; und
Ermitteln, ob beliebige zwei methylierte Basen in *cis* auf dem DNA-Molekül vorhanden sind;
**dadurch gekennzeichnet, dass** das im Wesentlichen isolierte DNA-Molekül mit einem physikalischen Verfahren isoliert wird; und wobei das im Wesentlichen isolierte DNA-Molekül aus einem einzelnen Chromosom oder einem Chromatid oder einem Fragment davon besteht.

2. Verfahren nach Anspruch 1, wobei das einzelne Chromosom oder das Chromatid oder Fragment davon durch laservermittelte Dissektion isoliert wird.

3. Verfahren nach Anspruch 1 oder 2, wobei das DNA-Molekül in einer Diploidzelle vorhanden ist oder von einer solchen gewonnen wird.

4. Verfahren nach einem der Ansprüche 1 bis 3, wobei das Analysieren das Ermitteln von Methylierung eines Dinukleotid-CpG beinhaltet.

5. Verfahren nach Anspruch 4 oder Anspruch 5, wobei das Analysieren ein Verfahren beinhaltet, das ausgewählt ist aus der Gruppe bestehend aus DNA-Sequenzierung mittels Bisulfitbehandlung, genomischem Restriktionslandmarken-Scanning, methylierungssensitiver willkürlich geprimter PCR, Southern-Analyse mit einem methylierungssensitiven Restriktionsenzym, methylierungsspezifischer PCR, Restriktionsenzymverdau von PCR-Produkten, amplifiziert von Bisulfit-konvertierter DNA, und Kombinationen davon.

6. Verfahren nach Anspruch 5, wobei das Analysieren, wo es DNA-Sequenzierung mit Bisulfitbehandlung beinhaltet, Folgendes beinhaltet:
(a) Umsetzen der DNA mit Natriumbisulfit zum Konvertieren von unmethylierten Cytosinresten in Uracilreste, während eventuelle 5-Methylcytosin-Reste unverändert gelassen werden, um eine exponierte Bisulfit-konvertierte DNA-Probe mit Bindungsstellen für Primer zu erzeugen, die für die Bisulfit-konvertierte DNA-Probe spezifisch sind;
(b) Ausführen eines PCR-Amplifikationsverfahrens mit oberstrang- oder unterstrangspezifischen Primern;
(c) Isolieren der PCR-Amplifikationsprodukte;
(d) Durchführen einer Primerverlängerungsreaktion mit einem Ms-SNuPE-Primer, dNTPs und Taq-Polymerase, wobei der Ms-SNuPE-Primer eine Primer-Sequenz mit einer Länge von 15-mer bis 22-mer umfasst, die zu der Bisulfit-konvertierten DNA-Probe komplementär ist und unmittelbar 5' vom Cytosin-Rest der zwei oder mehr zu untersuchenden CpG-Dinukleotidsequenzen terminiert; und
(e) Ermitteln des Methylierungszustands der zwei oder mehr CpG-Dinukleotidsequenzen durch Ermitteln der Identität der ersten Primer-verlängerten Base.

7. Verfahren nach Anspruch 6, wobei die dNTPs markiert sind und das Ermitteln der Identität der ersten Primer-verlängerten Base durch Einbeziehen der markierten dNTPs gemessen wird.

8. Verfahren nach einem der Ansprüche 1 bis 7, wobei die epigenetischen Informationen phänotypische Informationen über das Subjekt bereitstellen können, von dem die biologische Probe gewonnen wurde.

9. Verfahren nach Anspruch 8, wobei die phänotypischen Informationen ausgewählt werden aus der Gruppe bestehend aus An- oder Abwesenheit einer Krankheit, eines Zustands oder einer Störung; oder einer Prädisposition für eine Krankheit, einen Zustand oder eine Störung.

## Revendications

1. Procédé permettant d'obtenir l'information épigénétique concernant un échantillon biologique prélevé chez un sujet humain, animal ou végétal, le procédé comprenant l'étape consistant à :
analyser l'échantillon biologique, l'échantillon contenant une molécule d'ADN essentiellement isolée, en :
déterminant si deux ou plusieurs bases méthylées sont présentes ou absentes dans la molécule d'ADN, la présence ou l'absence des bases méthylées étant capable de moduler l'expression de la molécule d'ADN *in vivo* ; et
déterminant si deux bases méthylées quelconques sont présentes en *cis* sur la molécule d'ADN ;
**caractérisé en ce que** la molécule d'ADN essentiellement isolée est isolée en utilisant une méthode physique ; la molécule d'ADN essentiellement isolée consistant en un chromosome unique ou un chromatide ou en un fragment de celui-ci.

2. Procédé selon la revendication 1, dans lequel le chromosome unique ou le chromatide ou le fragment de celui-ci est isolé par microdissection par capture laser.

3. Procédé selon la revendication 1 ou 2, dans lequel la molécule d'ADN est présente dans ou obtenue à partir d'une cellule diploïde.

4. Procédé selon l'une quelconque des revendications 1 à 3, dans lequel l'analyse inclut la détermination de la méthylation d'un dinucléotide CpG.

5. Procédé selon la revendication 4 ou la revendication 5, dans lequel l'analyse inclut une méthode sélectionnée dans le groupe consistant en les suivantes : séquençage de l'ADN par traitement au bisulfite, technique RLGS (pour *restriction landmark genomic scanning*), PCR à amorce arbitraire sensible à la méthylation, analyse par Southern blot utilisant une enzyme de restriction sensible à la méthylation, PCR spécifique à la méthylation, digestion, par des enzymes de restriction, des produits de PCR amplifiés à partir de l'ADN converti au bisulfite et combinaisons de celles-ci.

6. Procédé selon la revendication 5, dans lequel quand l'analyse inclut un séquençage de l'ADN par traitement au bisulfite, l'analyse comprend :
(a) la mise en réaction de l'ADN avec du bisulfite de sodium pour convertir les résidus cytosines non méthylés en résidus uraciles tout en laissant tout résidu 5-méthylcytosine inchangé pour créer un échantillon d'ADN converti au bisulfite exposé ayant des sites de liaison pour des amorces spécifiques à l'échantillon d'ADN converti au bisulfite ;
(b) la réalisation d'une procédure d'amplification par PCR en utilisant le brin du haut ou le brin du bas d'amorces spécifiques ;
(c) l'isolement des produits d'amplification par PCR ;
(d) la réalisation d'une réaction d'extension de l'amorce en utilisant une amorce Ms-SNuPE, des dNTP et une Taq polymérase, l'amorce Ms-SNuPE comprenant une séquence d'amorçage de 15 mer à 22 mer de long qui est complémentaire à l'échantillon d'ADN converti au bisulfite et qui se termine immédiatement en 5' du résidu cytosine des deux ou plusieurs séquences à dinucléotides CpG à analyser ; et
(e) la mesure de l'état de méthylation des deux ou plusieurs séquences à dinucléotides CpG par une détermination de l'identité de la première base de l'extension d'amorce.

7. Procédé de la revendication 6, dans lequel les dNTP sont marqués et dans lequel la détermination de l'identité de la première base de l'extension d'amorce est mesurée par incorporation des dNTP marqués.

8. Procédé selon l'une quelconque des revendications 1 à 7, dans lequel l'information épigénétique est capable de fournir des informations phénotypiques concernant le sujet chez lequel l'échantillon biologique a été obtenu.

9. Procédé selon la revendication 8, dans lequel les informations phénotypiques sont sélectionnées dans le groupe consistant en les suivantes : présence ou absence d'une maladie, d'un état pathologique ou d'une affection ; ou prédisposition à une maladie, un état pathologique ou une affection.
